# EUROPÄISCHE PATENTSCHRIFT

(11) **EP 1 480 945 B1**
(45) Veröffentlichungstag und Bekanntmachung des Hinweises auf die Patenterteilung: **14.09.2005**
(21) Anmeldenummer: 03742928.9
(22) Anmeldetag: 06.02.2003
(51) Int. Cl.: C07C 249/12, C07C 251/38

(54) **VERFAHREN ZUR HERSTELLUNG VON 2-CHLORMETHYLPHENYLESSIGSÄUREDERIVATEN**
METHOD FOR PRODUCING 2-CHLOROMETHYLPHENYL ACETIC ACID DERIVATIVES
PROCEDE DE FABRICATION DE DERIVES D'ACIDE 2-CHLOROMETHYLPHENYLACETIQUE

(30) Priorität: 26.02.2002 DE 10208029
(43) Veröffentlichungstag der Anmeldung: 01.12.2004
(73) Patentinhaber: BASF Aktiengesellschaft, 67056 Ludwigshafen (DE)
(72) Erfinder: MAYER, Guido, 67161 Gönnheim (DE); CULLMANN, Oliver, 64646 Heppenheim (DE); WOLF, Bernd, 67136 Fussgönheim (DE); KEIL, Michael, 67251 Freinsheim (DE)
(86) Internationale Anmeldenummer: PCT/EP2003/001160
(87) Internationale Veröffentlichungsnummer: WO 2003/072538

(56) Entgegenhaltungen:
- WO-A-97/21686
- JEMPTY, THOMAS C. ET AL: "Iron trichloride/silicon dioxide reacts as oxidant or Lewis aci with phenol ethers" JOURNAL OF ORGANIC CHEMISTRY ( 1981 ), 46(22), 4545-51 , XP002241982 in der Anmeldung erwähnt

## Beschreibung

Die vorliegende Erfindung betrifft ein Verfahren zur Herstellung von 2-Chlormethylphenylessigsäurederivaten der Formel I, 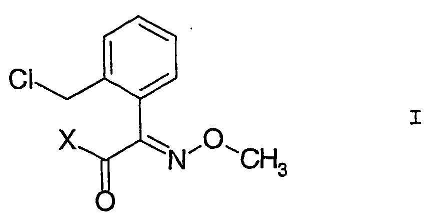 in der X für C₁-C₄-Alkoxy oder Methylamino steht, durch Etherspaltung von Verbindungen der Formel II, 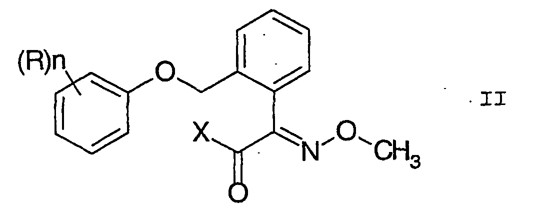 in der R C₁-C₄-Alkyl, C₁-C₄-Alkoxy, C₁-C₂-Halogenalkyl, C₁-C₄-Alkylcarbonyl, C₁-C₄-Alkylcarbonyloxy, Halogen, Nitro oder Cyano bedeutet und X die obengenannte Bedeutung besitzt.

Aus J. Chem. Research (S) 232-3 (1985) und aus J. Org. Chem. 64, 4545 (1981) sind Methoden zur Spaltung von Benzylethern in Gegenwart von speziellen Lewissäuren wie Natriumiodid/Bortrifluorid bzw. Eisen(III)chlorid auf Kieselgel bekannt. Die Lewissäuren kommen hierbei in mehr als stöchiometrischen Mengen zum Einsatz, was die Verfahren unwirtschaftlich macht.

In Synlett (10), 1575-6 (1999) wird ein Verfahren zur Spaltung von 4-Nitrobenzylethern in Gegenwart von Indium und wässrigem Ammoniumchlorid beschrieben. Indium wird hierbei in einem Überschuß von mehr als 8 Äquivalenten bezogen auf den zu spaltenden Ether eingesetzt.

Ein Verfahren zur Herstellung von 2-Chlormethylphenylessigsäurederivaten der Formel I durch Spaltung der entsprechenden Benzylether II ist in WO-A 97/21686 beschrieben. Der Benzylether II wird hierin mit einem mehrfachen molaren Überschuß an Bortrichlorid versetzt.

Die im Stand der Technik bekannten Verfahren zeichnen sich durch den mehr als stöchiometrischen Einsatz von Lewissäuren aus. Die verwendeten Lewissäuren sind zudem problematisch in der Handhabung und größtenteils äußerst korrosiv.

Es bestand die Aufgabe, ein mit hoher Ausbeute und Selektivität durchführbares katalytisches Verfahren zur Herstellung von 2-Chlormethylphenylessigsäurederivaten der Formel I aus den entsprechenden Benzylethern bereitzustellen, das die obengenannten Nachteile nicht aufweist. Weiterhin war darauf zu achten, dass der Benzylether II mit hoher Selektivität gespalten wird, d.h. die Methoxyiminophenylglyoxylsäure-Einheit in der Zielverbindung I sollte erhalten bleiben.

Diese Aufgabe wird dadurch gelöst, dass die Etherspaltung in Gegenwart von Chlorwasserstoff und einem inerten Lösungsmittel stattfindet, und dem Reaktionsgemisch ein Katalysator ausgewählt aus der Gruppe: Eisen, Indium oder deren Halogenide, Oxide oder Triflate zugesetzt wird.

Der Chlorwasserstoff wird in der Regel gasförmig in das Reaktionsgemisch eingeleitet. Es ist jedoch auch möglich den Chlorwasserstoff einzukondensieren. Im allgemeinen wird der Chlorwasserstoff in einem Molverhältnis in Bezug auf den Benzylether von 1 bis 25, vorzugsweise 1 bis 10 und insbesondere bevorzugt 3 bis 5 Moläquivalenten eingesetzt.

Als Katalysator dienen Lewissäuren ausgewählt aus der Gruppe: Eisen, Indium oder deren Halogenide, Oxide oder Triflate. Bevorzugt sind die Katalysatoren: Eisen und Indium(III)chlorid sowie insbesondere Eisen(III)oxid und Eisen(III)chlorid. Der Katalysator wird in einer Konzentration von 0,001 bis 0,5 und vorzugsweise 0,01 bis 0,2 Moläquivalenten eingesetzt.

Als Lösungsmittel kommen aromatische (halogenierte) Kohlenwasserstoffe wie z.B. Benzol, Toluol, Xylol, Chlorbenzol, Dichlorbenzol, Brombehzol und Benzotrifluorid; aliphatische (halogenierte) Kohlenwasserstoffe wie z.B. Pentan, Heptan, Dichlormethan, Chloroform, 1,2-Dichlorethan oder Tetrachlorkohlenstoff; cycloaliphatische Kohlenwasserstoffe wie z.B Cyclohexan oder Cyclopentan; Ether wie z.B. Dimethoxyethan, Diethylether, Di-isopropylether, Ester wie z.B. Essigsäureethylester oder Essigsäurebutylester in Frage. Es können auch Gemische dieser Lösungsmittel eingesetzt werden.

Bevorzugte Lösungsmittel sind aromatische (halogenierte) Kohlenwasserstoffe und aliphatische (halogenierte) Kohlenwasserstoffe.

Unter Umständen kann es von Vorteil sein dem Reaktionsgemisch Lewisbasen wie z.B. Pyridin, N,N-Dimethylanilin oder Ethanthiol und/oder weitere Hilfsstoffe wie Trimethylsilylchlorid zuzusetzen.

Weiterhin kann es von Vorteil sein in einem Zweiphasensystem in Gegenwart eines Phasentransferkatalysators wie z.B. Tetrabutylammoniumchlorid, Tetrahexylammoniumchlorid, Tetrabutylphosphoniumchlorid, Bis (triphenylphosphoranyliden) ammoniumchlorid, Trimethylbenzylammoniumchlorid, Triethylbenzylammoniumchlorid oder Triphenylbenzylammoniumchlorid zu arbeiten.

Die Reaktiontemperatur beträgt üblicherweise 0 bis 100°C und vorzugsweise 30 bis 70°C. Der Reaktionsdruck beläuft sich üblicherweise auf 0 bis 6 bar. Vorzugsweise wird die Reaktion drucklos durchgeführt.

Weiterhin ist es vorteilhaft, die Etherspaltung unter Schutzgasatmosphäre auszuführen.

Als Ausgangsstoffe für die Etherspaltung kommen die eingangs erwähnten Benzylether II in Frage. Sie sind nach literaturüblichen Methoden zugänglich (EP-A 253 213, EP-A 254 426, EP-A 398 692 oder EP-A 477 631). Insbesondere eignen sich die am Markt gängigen Pflanzenschutzmittel wie beispielsweise 2-Methoxyimino-2-[(2-methylphenyloxymethyl)phenyl]essigsäuremethylester (Kresoxim-methyl, EP-A 253 213).

Nach der Etherspaltung wird das Reaktionsgemisch in der Regel extraktiv aufgearbeitet. Verunreinigungen mit Katalysator lassen sich beispielsweise durch Extraktion mit wäßriger Mineralsäure wie Salzsäure entfernen. Das abgespaltene Phenol läßt sich vorteilhaft durch Extraktion mit wäßriger Alkali wie Natronlauge entfernen.

Das erhaltene 2-Chlormethylphenylessigsäurederivat kann direkt im inerten Lösungsmittel gelöst oder nach destillativem Entfernen des Lösungsmittels als Schmelze weiterverarbeitet werden.

Das Rohprodukt läßt sich durch Umkristallisieren in Alkoholen wie Methanol, Ethanol, n-Butanol oder Gemischen davon oder Gemischen von Alkoholen und Dimethylformamid weiter reinigen. Weiterhin läßt sich das Rohprodukt durch Schmelzkristallation reinigen.

### Verfahrensbeispiele

### Beispiel 1

In 150 ml Chlorbenzol wurden 7,5 g (24 mmol) Kresoxim-methyl gelöst. Anschließend wurden 0,32 g (2,4 mmol) Eisen(III)chlorid zugegeben und danach innerhalb von 1 h, während der Aufheizphase auf 50°C, 2,6 g (72 mmol) Chlorwasserstoff eingegast. Unter Rühren wurde die Reaktionsmischung weitere 2 Stunden auf 50°C gehalten und anschließend der Umsatz mittels HPLC kontrolliert. Nach beendeter Reaktion wurde die Reaktionslösung abgekühlt und mit 10 ml Methanol versetzt. Die Reaktionsmischung wurde zunächst mit Salzsäure und anschließend mit Natronlauge extrahiert. Die organische Phase wurde neutral gewaschen und anschließend vom Lösugsmittel befreit. Die Ausbeute an 2-Methoxyimino-2-[(2-chlormethyl)phenyl]essigsäuremethylester betrug 75 %.

### Beispiel 2

In 150 ml Toluol werden 7,5 g (24 mmol) Kresoxim-methyl gelöst. Anschließend wurden 0,53 g (2,4 mmol) Indium(III)chlorid zugegeben und innerhalb von 1 h, während der Aufheizphase auf 40°C, 2,6 g (72 mmol) Chlorwasserstoff eingegast. Unter Rühren wurde die Reaktionsmischung weitere 4 Stunden bei 40 °C gehalten und anschließend wie in Beispiel 1 aufgearbeitet. Die Ausbeute an 2-Methoxyimino-2-[(2-chlormethyl)phenyl]essigsäuremethylester betrug 80 %.

### Beispiel 3

Die Etherspaltung erfolgte analog Beispiel 1, in 150 ml 1,2-Dichlorethan. Es wurden innerhalb von 1 h während der Aufheizphase auf 100°C 4,1 g (112 mmol) Chlorwasserstoff eingegast und die Reaktionsmischung weitere 5 Stunden auf 100 °C gehalten. Die Ausbeute am Wertprodukt betrug 80 %.

### Vergleichsbeispiele

### Beispiel 4

In 150 ml Toluol wurden 7,5 g (24 mmol) Kresoxim-methyl gelöst. Anschließend wurden 0,32 g (2,4 mmol)Aluminiumchlorid zugegeben und danach wurden innerhalb von 1 h während der Aufheizphase auf 100°C 2,6 g (72 mmol) Chlorwasserstoff eingegast. Unter Rühren wurde die Reaktionsmischung weitere 2 Stunden auf 100°C gehalten und anschließend wie in Beispiel 1 aufgearbeitet. Die Ausbeute am Wertprodukt betrug 30 %.

### Beispiel 5

In 150 ml 1,2-Dichlorethan wurden 7,5 g (24 mmol) Kresoxim-methyl gelöst. Anschließend wurden 0,63 g (2,4 mmol) Zinntetrachlorid zugegeben und innerhalb von 1 h, während der Aufheizphase auf 85°C, 2,6 g (72 mmol) Chlorwasserstoff eingegast. Unter Rühren wurde die Reaktionsmischung weitere 4 Stunden auf 85°C gehalten und anschließend wie in Beispiel 1 aufgearbeitet. Die Ausbeute am Wertprodukt betrug 30 %.

## Patentansprüche

1. Verfahren zur Herstellung von 2-Chlormethyl-phenylessigsäurederivaten der Formel I, 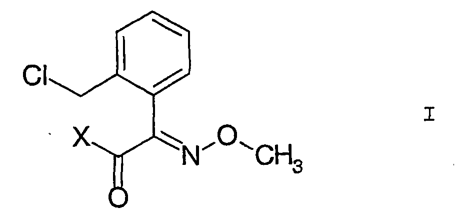 in der X für C₁-C₄-Alkoxy oder Methylamino steht, durch Etherspaltung von Verbindungen der Formel II, 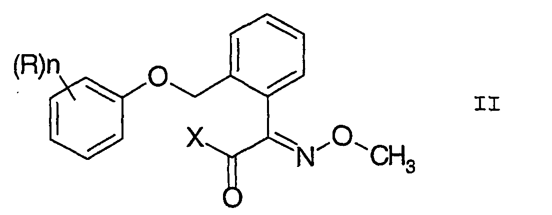 in der R C₁-C₄-Alkyl, C₁-C₄-Alkoxy, C₁-C₂-Halogenalkyl, C₁-C₄-Alkylcarbonyl, C₁-C₄-Alkylcarbonyloxy, Halogen, Nitro oder Cyano bedeutet und X die obengenannte Bedeutung besitzt, **dadurch gekennzeichnet, dass** die Umsetzung in Gegenwart von Chlorwasserstoff und einem inerten Lösungsmittel stattfindet, und dem Reaktionsgemisch ein Katalysator ausgewählt aus der Gruppe: Eisen, Indium oder deren Halogenide, Oxide oder Triflate zugesetzt wird.

2. Verfahren nach Anspruch 1, **dadurch gekennzeichnet, dass** als Katalysator Eisen(III)chlorid eingesetzt wird.

3. Verfahren nach den Ansprüchen 1 und 2, **dadurch gekennzeichnet, dass** der Katalysator in einer Konzentration von 0,001 bis 0,5 Moläquivalenten eingesetzt wird.

4. Verfahren nach Anspruch 1 bis 3, **dadurch gekennzeichnet, dass** 1 bis 25 Moläquivalente Chlorwasserstoff eingesetzt werden.

5. Verfahren nach Anspruch 1 bis 4, **dadurch gekennzeichnet, dass** als inertes Lösungsmittel ein aromatischer oder aliphatischer gegebenenfalls halogenierter Kohlenwasserstoff eingesetzt wird.

## Claims

1. A process for preparing 2-(chloromethyl)phenylacetic acid derivatives of the formula I, 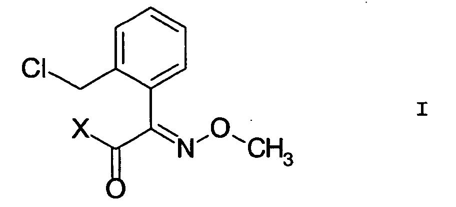 where X is C₁-C₄-alkoxy or methylamino, by ether cleavage of compounds of the formula II, 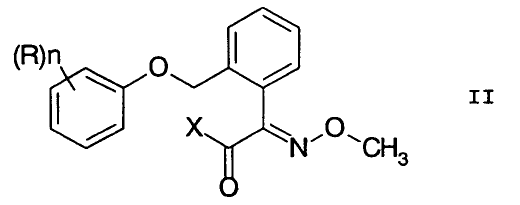 where R is C₁-C₄-alkyl, C₁-C₄-alkoxy, C₁-C₂-haloalkyl, C₁-C₄-alkylcarbonyl, C₁-C₄-alkylcarbonyloxy, halogen, nitro or cyano and X is as defined above, which comprises carrying out the reaction in the presence of hydrogen chloride and an inert solvent, and adding a catalyst to the reaction mixture selected from the group consisting of iron, indium and halides, oxides and triflates thereof.

2. A process as claimed in claim 1, wherein the catalyst used is iron(III) chloride.

3. A process as claimed in claim 1 or 2, wherein the catalyst is used in a concentration of from 0.001 to 0.5 mol equivalents.

4. A process as claimed in any of claims 1 to 3, wherein from 1 to 25 mol equivalents of hydrogen chloride are used.

5. A process as claimed in any of claims 1 to 4, wherein the inert solvent used is an aromatic or aliphatic, optionally halogenated hydrocarbon.

## Revendications

1. Procédé de préparation de dérivés d'acide 2-chlorométhyl-phénylacétique de la formule I 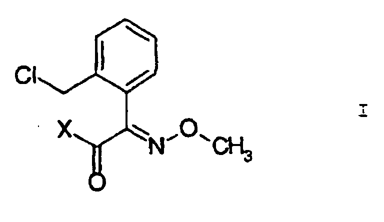 dans laquelle X représente un groupe alcoxy en C₁-C₄ ou méthylamino, par coupure d'éther de composés de la formule II 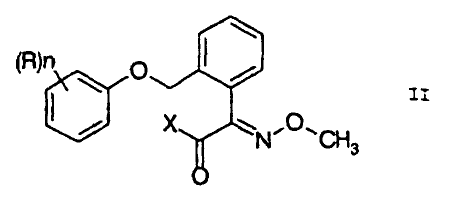 dans laquelle R représente un groupe alkyle en C₁-C₄, alcoxy en C₁-C₄, halogénoalkyle en C₁-C₂, alkylcarbonyle en C₁-C₄, alkylcarbonyloxy en C₁-C₄, halogène, nitro ou cyano et X présente la signification précitée, **caractérisé en ce que** la réaction a lieu en présence de chlorure d'hydrogène et d'un solvant inerte et **en ce qu'**on ajoute au mélange réactionnel un catalyseur choisi parmi le groupe : fer, indium ou leurs halogénures, oxydes ou triflates.

2. Procédé suivant la revendication 1, **caractérisé en ce que**, comme catalyseur, on met en oeuvre du chlorure de fer (III).

3. Procédé suivant les revendications 1 et 2, **caractérisé en ce que** le catalyseur est mis en oeuvre en une concentration de 0,001 à 0,5 équivalents molaires.

4. Procédé suivant une revendication 1 à 3, **caractérisé en ce qu'**on met en oeuvre 1 à 25 équivalents molaires de chlorure d'hydrogène.

5. Procédé suivant une revendication 1 à 4, **caractérisé en ce que**, comme solvant inerte, on met en oeuvre un hydrocarbure aromatique ou aliphatique, éventuellement halogéné.
